# EUROPEAN PATENT APPLICATION

(11) **EP 4 491 152 A1**
(43) Date of publication of application: **15.01.2025**
(21) Application number: 23766115.2
(22) Date of filing: 09.03.2023
(51) Int. Cl.: A61F 2/07, A61L 31/02, A61L 31/04, A61L 31/06

(54) **METHOD FOR MANUFACTURING COVERED STENT, COVERED STENT, AND COVERED STENT DELIVERY SYSTEM**

(30) Priority: 09.03.2022 CN 202210232680
(71) Applicant: Jiangsu Polylive Medtech Co., Ltd., Nantong, Jiangsu 226400 (CN)
(72) Inventor: GU, Fan, Nantong, Jiangsu 226400 (CN); SHENG, Zeyuan, Nantong, Jiangsu 226400 (CN)
(74) Representative: Zaboliene, Reda
(86) International application number: PCT/CN2023/080633
(87) International publication number: WO 2023/169538

(57) **Abstract**

A method for manufacturing a covered stent (10), comprising: providing an inner membrane (11), and sleeving the inner membrane (11) on a core rod; providing a perforated mold (20), and fixing the perforated mold (20) onto the inner membrane (11), wherein the perforated mold (20) is provided with a plurality of through holes (101) for exposing the inner membrane (11); providing an adhesive liquid, and arranging the adhesive liquid in each of the through holes (101) to form an adhesive body (14) in each of the through holes (101); removing the perforated mold (20) to leave the adhesive bodies (14) arranged in a lattice pattern on a surface of the inner membrane (11); providing a vascular stent (13), wherein the vascular stent (13) comprises a plurality of wavy ribs (131), and adjacent two ribs (131) are arranged in mirror symmetry, and sleeving the vascular stent (13) on the inner membrane (11), such that a plurality of adhesive bodies (14) are distributed on a symmetry line of adjacent two ribs (131); providing an outer membrane (12), and covering the outer membrane (12) on the vascular stent (13); providing a heat shrinkable device, and sleeving the heat shrinkable device on the outer membrane (12); and heating the overall structure on the core rod, such that the adhesive bodies (14) are fixedly connected between the inner membrane (11) and the outer membrane (12). Further provided are a covered stent (10) and a covered stent delivery system (30).

## Description

### Technical Field

The present invention relates to the field of medical device technology, and in particular to a method for manufacturing a covered stent, a covered stent, and a covered stent delivery system.

### Description of Related Art

Vascular diseases mainly refer to atherosclerosis, inflammatory vascular diseases, functional vascular diseases, true tumor diseases of blood vessels, etc. Among them, atherosclerosis is the most common. For vascular diseases, stent is currently the most widely used minimally invasive interventional surgical treatment medical device. Interventional surgery has the advantages of minimally invasive therapy, such as small incision, mild pain, and fast recovery. With the support of modern medical imaging technology, vascular stents, by expanding to a certain diameter or using the principle of the balloon dilation, are guided by guide wires to the site of the narrowed segment of the lesion, achieving the purposes of supporting the narrowed and occluded segment of the vessel, reducing vessel retraction, maintaining smooth blood flow in the blood cavity, and reshaping.

Generally speaking, stents are divided into self-expanding stents and balloon expandable stents. Self-expanding stents are relatively flexible and are generally used in long sections of lesions with low support requirements. Balloon expandable stents have higher support and accurate positioning, and are often used in short, eccentric, and particularly calcified lesions. From the perspective of structural forms, stents can be divided into bare stent platforms, drug-eluting stents, covered stents, etc. The three are used for different types of diseases. Generally speaking, for chronic stenosis lesions, bare stent platforms can be used as treatment methods. On this basis, drug-eluting stents can be used to further reduce restenosis rates and improve endothelialization speed. However, when the elasticity of the blood vessels in the affected area is severely insufficient, or when there is severe calcification, diffuse thrombosis, or even complete occlusion in the affected area, the use of bare stent platforms or drug-eluting stents for dilation may cause serious risks such as vascular tearing, vascular dissection, thrombus detachment, and thrombus displacement. Therefore, in recent years, covered stents have become the primary choice for treating this type of disease. Further, covered stents can also be used for anti-bleeding or protective treatments such as hemangioma, vascular dissection, and vascular perforation. From a clinical perspective, the first stage patency rate and target lesion reconstruction rate of covered stents are significantly higher than those of bare stents.

### BRIEF SUMMARY OF THE INVENTION

### Technical Problem

The membranes of the covered stent are mostly made of polymer membranes, and the most common material is expanded polytetrafluoroethylene. This material can achieve rapid endothelialization and reduce the risk of thrombosis when used for vascular implantation. However, due to its own material performance characteristics, ePTFE material also restricts its processing performance, making it difficult to prepare as a covered stent. Furthermore, the existing covered stents have problems such as poor bonding stability and poor elastic expansion.

### Technical Solution

In order to address the aforementioned shortcomings in the existing technology, the present invention aims to provide a method for manufacturing a covered stent to ensure the firmness and expansion elasticity of the membranes.

The present invention provides a method for manufacturing a covered stent, wherein the method for manufacturing a covered stent includes:
providing an inner membrane, and sleeving the inner membrane on a core rod;
providing a perforated mold, and fixing the perforated mold onto the inner membrane, wherein the perforated mold is provided with a plurality of through holes for exposing the inner membrane, and the plurality of through holes are arranged in a lattice pattern;
providing an adhesive liquid, and arranging the adhesive liquid in each of the through holes to form an adhesive body in each of the through holes;
removing the perforated mold to leave the adhesive bodies arranged in a lattice pattern on a surface of the inner membrane;
providing a vascular stent, wherein the vascular stent comprises a plurality of wavy ribs, and adjacent two ribs are arranged in mirror symmetry, and sleeving the vascular stent on the inner membrane, such that a plurality of adhesive bodies are distributed on a symmetry line of adjacent two ribs, or a plurality of adhesive bodies are distributed adjacent to a symmetry line of adjacent two ribs;
providing an outer membrane, and covering the outer membrane on the vascular stent;
providing a heat shrinkable device, and sleeving the heat shrinkable device on the outer membrane;
heating the overall structure on the core rod, such that the adhesive bodies are fixedly connected between the inner membrane and the outer membrane, and then removing the heat shrinkable device and the core rod to form a covered stent.

Optionally, before sleeving the inner membrane and/or the outer membrane on the core rod, the method includes:
performing a surface treatment on an outer surface of the inner membrane and an inner surface of the outer membrane, wherein the surface treatment includes any one or more than one of the following:
using a surface modifier containing boron and amino groups;
using an etching method with sodium naphthalene;
using a processing method with ArF laser;
using a chemical grafting method.

Optionally, the perforated mold is made of FEP membrane, steel strip, or steel pipe.

Optionally, the heat shrinkable device is a heat shrink tube or a heat treatment mold; the inner membrane and the outer membrane are ePTFE membranes.

Optionally, the adhesive liquid is a solution, a suspension, or an emulsion; the solute of the adhesive liquid includes granule or powder of polymer materials.

Optionally, the solvent of the adhesive liquid is a liquid that is volatile, has no chemical residue, and does not corrode the inner membrane and the outer membrane.

Optionally, the heat shrinkable device is a heat shrink tube or a heat treatment mold.

Optionally, the method of drying the adhesive liquid includes heating to dry or drying in the air, wherein the temperature of heating to dry is 60°C-300°C, and the drying time is 15-60 minutes.

Optionally, the temperature for heating the overall structure on the core rod is 200°C-300°C, and the time is 5-60 minutes.

Optionally, the inner membrane and the outer membrane are ePTFE membranes.

Optionally, the wavy ribs extend in a wave shape, a sawtooth shape, or a square wave shape.

Optionally, the rib includes a plurality of bending units connected to each other, and each bending unit includes a first rib section and a second rib section, which are arranged in mirror symmetry; when the vascular stent is sleeved on the inner membrane, a plurality of adhesive bodies are distributed on a symmetry line of the first rib section and the second rib section, or a plurality of adhesive bodies are distributed adjacent to a symmetry line of the first rib section and the second rib section.

Optionally, the inner membrane and/or the outer membrane is a tubular or sheet-like membrane.

Optionally, the vascular stent further includes a plurality of connecting bars connected between adjacent two ribs, the connecting bar is in a straight strip shape, a wave shape, a sawtooth shape, a square wave shape, a plate wave shape, or a symbol Ω.

The present invention further provides a covered stent, wherein the covered stent is manufactured using the method for manufacturing a covered stent as described above.

Optionally, the covered stent includes an inner membrane, an outer membrane, a vascular stent, and a plurality of adhesive bodies, the inner membrane is arranged on an inner side of the vascular stent, the outer membrane is arranged on an outer side of the vascular stent, the vascular stent is located between the inner membrane and the outer membrane, the plurality of adhesive bodies are arranged in a lattice pattern, one end of each adhesive body is fixed to the inner membrane, and the other end of each adhesive body is fixed to the outer membrane.

The present invention further provides a covered stent delivery system, wherein the covered stent delivery system is used to deliver the covered stent as described above into a blood vessel, the covered stent delivery system includes a connecting member, a wire guide tube, a liquid guide tube, a balloon, and a developing ring, a wire guide cavity and a liquid guide cavity are provided inside the connecting member, both the wire guide tube and the liquid guide tube are connected to the connecting member, the wire guide tube is communicated with the wire guide cavity, the liquid guide tube is communicated with the liquid guide cavity, the wire guide tube is inserted into the liquid guide tube, one end of the wire guide tube extends out from one end of the liquid guide tube, one end of the balloon is connected to the wire guide tube, the other end of the balloon is connected to the liquid guide tube, the liquid guide tube is communicated with the balloon, the developing ring is connected to the wire guide tube, and the covered stent is installed on the balloon.

### Beneficial Effects

The method for manufacturing a covered stent of the present invention adopts a lattice arrangement of the adhesive bodies to connect the inner membrane and the outer membrane. The adhesive bodies are distributed on the symmetrical line of adjacent two ribs, or the adhesive bodies are distributed adjacent to the symmetrical line of adjacent two ribs. The adhesive bodies are located far away from the ribs. The adhesive bodies are located at the positions having minimal influence to the expansion process of the inner membrane and the outer membrane. The adhesive bodies can more firmly connect the inner membrane with the outer membrane, making the shape of the overall membranes controllable, while ensuring the expansion elasticity of the material at the unconnected locations of the inner membrane and the outer membrane. Moreover, the covered stent manufactured by the manufacturing method further includes a plurality of connecting bars connected between adjacent two ribs. When the vascular stent expands, the connecting bars are compressed by adjacent two ribs, and the connecting bars can provide reverse support force to the adjacent ribs. Therefore, the covered stent has the function of reducing axial shortening rate.

### BRIEF DESCRIPTION OF THE DRAWINGS

FIG. 1 is a schematic diagram of the method for manufacturing a covered stent according to the first embodiment of the present invention.
FIG. 2 is a partial schematic diagram of the method for manufacturing a covered stent of the present invention when fixing the perforated mold on the inner membrane.
FIG. 3 is a schematic diagram of the covered stent of the present invention after partially removing the outer membrane.
FIG. 4 is a schematic cross-sectional view of the covered stent shown in FIG. 3.
FIG. 5 is a schematic diagram of the unfolded state of the vascular stent and the adhesive bodies according to the first embodiment of the present invention.
FIG. 6 is a schematic diagram of the unfolded state of the vascular stent and the adhesive bodies according to another embodiment of the present invention.
FIG. 7 is a structural schematic diagram of the connecting bar according to the second embodiment of the present invention.
FIG. 8 is a structural schematic diagram of the connecting bar according to the third embodiment of the present invention.
FIG. 9 is a schematic diagram of the unfolded state of the vascular stent and the adhesive bodies according to the fourth embodiment of the present invention.
FIG. 10 is an enlarged schematic diagram of the connecting bar shown in FIG. 9.
FIG. 11 is a structural schematic diagram of the covered stent delivery system according to the fifth embodiment of the present invention.

### DETAILED DESCRIPTION OF THE EMBODIMENTS

The following specific embodiments illustrate the implementation of the present invention, and those familiar with this technology can easily understand other advantages and effects of the present invention from the content disclosed in this specification.

In the following description, reference is made to the accompanying drawings, which illustrate several embodiments of the present invention. It should be understood that other embodiments may also be used, and mechanical composition, structure, electrical, and operational changes may be made without departing from the spirit and scope of the present invention. The following detailed description should not be considered limiting, and the terms used here are only for describing specific embodiments and not intended to limit the present invention.

Although the terms "first", "second", etc. are used in this description to describe various elements in some instances, these elements should not be limited by these terms. These terms are only used to distinguish one element from another.

Further, as used in this description, the singular forms "an", "one", and "the" are intended to include the plural form as well, unless the context indicates otherwise. It should be further understood that the terms "comprise" and "include" indicate the existence of features, steps, operations, elements, components, items, categories, and/or groups, but do not exclude the existence, appearance, or addition of one or more other features, steps, operations, elements, components, items, categories, and/or groups. The terms "or" and "and/or "used here are interpreted as inclusive or imply any one or any combination. Therefore, "A, B, or C" or "A, B, and/or C" means any of the following: A; B; C; A and B; A and C; B and C; A, B and C. Exceptions to this definition only occur when a combination of components, functions, steps, or operations are inherently mutually exclusive in certain ways.

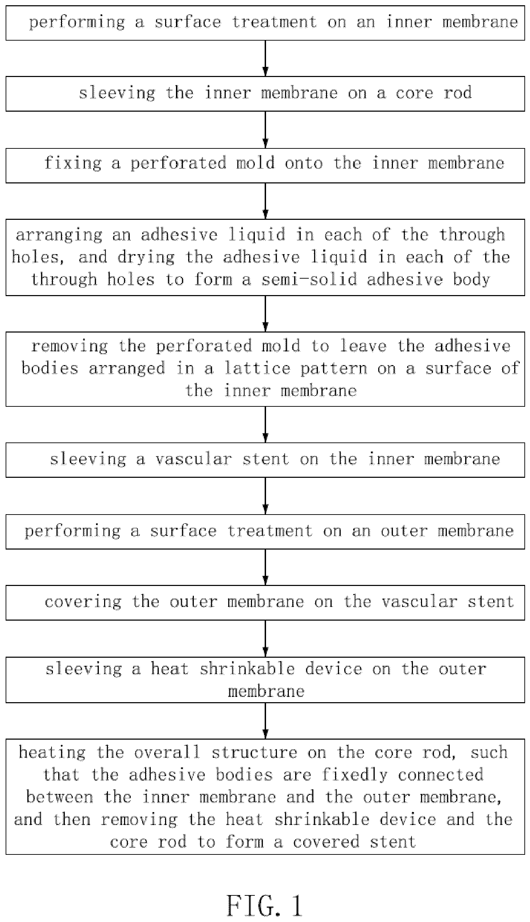

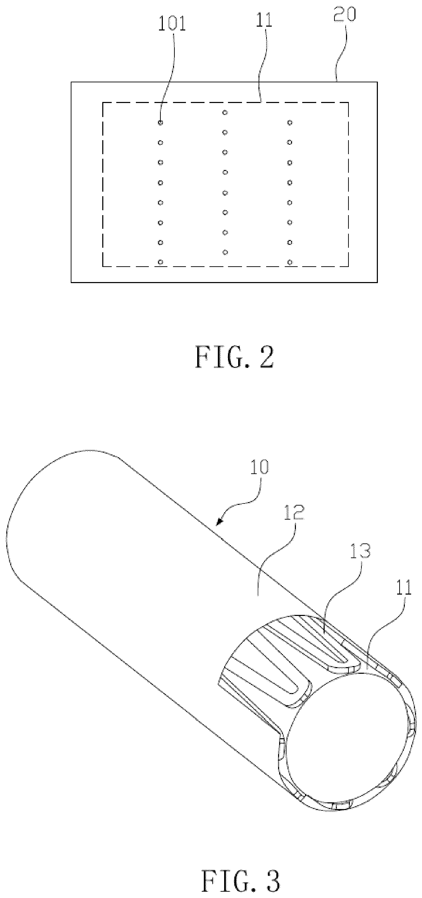

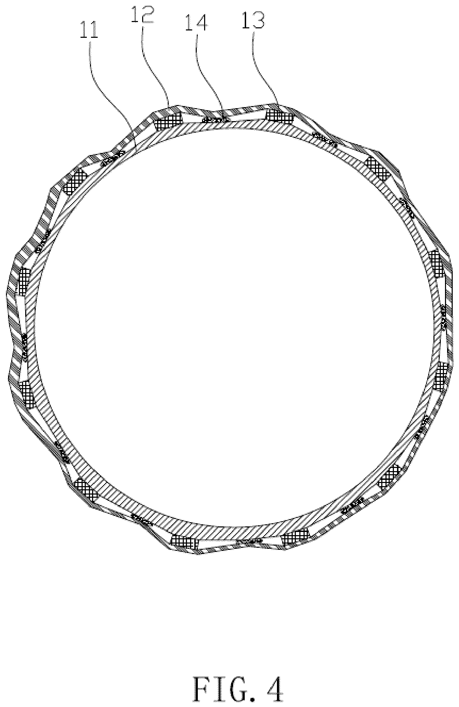

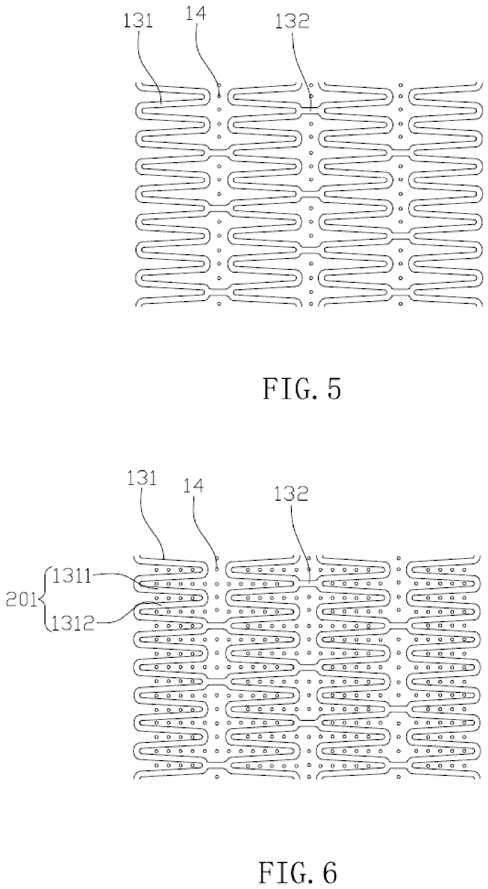

FIG. 1 is a schematic diagram of the method for manufacturing a covered stent according to the first embodiment of the present invention. FIG. 2 is a partial schematic diagram of the method for manufacturing a covered stent of the present invention when fixing the perforated mold on the inner membrane. FIG. 3 is a schematic diagram of the covered stent of the present invention after partially removing the outer membrane. FIG. 4 is a schematic cross-sectional view of the covered stent shown in FIG. 3. FIG. 5 is a schematic diagram of the unfolded state of the vascular stent and the adhesive bodies according to the first embodiment of the present invention. Please refer to FIG. 1 to FIG. 5, the method for manufacturing a covered stent includes:
providing an inner membrane 11, and sleeving the inner membrane 11 on a core rod;
providing a perforated mold 20, and fixing the perforated mold 20 onto the inner membrane 11, wherein the perforated mold 20 is provided with a plurality of through holes 101 for exposing the inner membrane 11, and the plurality of through holes 101 are arranged in a lattice pattern, as shown in FIG. 2;
providing an adhesive liquid, and arranging the adhesive liquid in each of the through holes 101 to form an adhesive body 14 in each of the through holes 101, for example, the adhesive liquid in each of the through holes 101 is dried or naturally evaporated to form a semi-solid adhesive body 14;
removing the perforated mold 20 to leave the adhesive bodies 14 arranged in a lattice pattern on a surface of the inner membrane 11;
providing a vascular stent 13, wherein the vascular stent 13 includes a plurality of wavy ribs 131, and adjacent two ribs 131 are arranged in mirror symmetry, and sleeving the vascular stent 13 on the inner membrane 11, such that a plurality of adhesive bodies 14 are distributed on a symmetry line of adjacent two ribs 131, or a plurality of adhesive bodies 14 are distributed adjacent to a symmetry line of adjacent two ribs 131; wherein the core rod is used to provide support for the inner membrane 11 and the vascular stent 13;
providing an outer membrane 12, and covering the outer membrane 12 on the vascular stent 13;
providing a heat shrinkable device, and sleeving the heat shrinkable device on the outer membrane 12;
heating the overall structure on the core rod, such that the adhesive bodies 14 are fixedly connected between the inner membrane 11 and the outer membrane 12, and then removing the heat shrinkable device and the core rod to form a covered stent 10, as shown in FIG. 3. In this embodiment, the heating temperature should be higher than the glass transition temperature of the adhesive bodies 14, such that the adhesive bodies 14 reach the high elastic state or molten state of the material.

The method for manufacturing a covered stent of the present invention adopts a lattice arrangement of the adhesive bodies 14 to connect the inner membrane 11 and the outer membrane 12. The adhesive bodies 14 are distributed on the symmetrical line of adjacent two ribs 131, or the adhesive bodies 14 are distributed adjacent to the symmetrical line of adjacent two ribs 131. The adhesive bodies 14 are located away from the ribs 131. The adhesive bodies 14 are located at the positions having minimal influence to the expansion process of the inner membrane 11 and the outer membrane 12. The adhesive bodies 14 can more firmly connect the inner membrane 11 with the outer membrane 12, making the shape of the overall membranes controllable, while ensuring the expansion elasticity of the material at the unconnected locations of the inner membrane 11 and the outer membrane 12.

Optionally, before sleeving the inner membrane 11 and/or the outer membrane 12 on the core rod, the method includes:
performing a surface treatment on an outer surface of the inner membrane 11 and an inner surface of the outer membrane 12, wherein the surface treatment includes any one or more than one of the following:
using a surface modifier containing boron and amino groups;
using an etching method with sodium naphthalene;
using a processing method with ArF laser;
using a chemical grafting method. In this embodiment, by performing surface treatment on the surface of the inner membrane 11 and/or the outer membrane 12, it can be ensured that the adhesive bodies 14 are more firmly connected to the inner membrane 11 and the outer membrane 12. It should be noted that the above methods are only specific examples, and the practical applications can include, but are not limited to, using the above methods for processing.

Optionally, the perforated mold 20 is made of FEP membrane, steel strip, or steel pipe. The through holes 101 on the perforated mold 20 can be made by laser engraving or electric drilling, but it is not limited to this.

Optionally, the adhesive liquid can be a solution, a suspension or an emulsion; the solute of the adhesive liquid includes granule or powder of polymer materials; the solvent of the adhesive liquid is a liquid that is volatile, has no chemical residue, and does not corrode the inner membrane 11 and the outer membrane 12, or an organic solvent, or a solvent classified by the FDA as having low toxicity potential (Class III solvent). In this embodiment, the organic solvent includes ethanol, tetrahydrofuran, isopropanol, or dimethyl sulfoxide.

Optionally, the method of preparing the solution includes using a granule of polyester fiber, polyurethane, or other thermoplastic elastomers to prepare a solution.

Optionally, the method of preparing the suspension or emulsion includes using ETFE, PFA, PVDF, FEP and other fluoropolymers to prepare a suspension or emulsion.

Optionally, the adhesive liquid is applied to the perforated mold 20 through methods such as coating, spraying, etc. By controlling the concentration of the adhesive liquid and the spraying process, it is ensured that there is sufficient adhesive liquid in the through holes 101 of the perforated mold 20, and there is no adhesive liquid in other locations. For example, the emulsion and the solvent are prepared into an emulsion in a mass ratio of 1:5-1:20, and the emulsion is dynamically stable for at least 10-60 minutes. After the spacing between each pipette dropper is adjusted according to a size of the perforated mold 20, the pipette droppers are used to release the emulsion until the emulsion fills the through holes 101, and then the pipette droppers are removed.

In other embodiments, the emulsion and the solvent can be prepared into an emulsion in a mass ratio of 1:10-1:30, and the emulsion is dynamically stable for at least 10-60 minutes. The prepared emulsion is put into a spray coater or brush coater for evenly applying for 30 seconds or more, waiting for the solvent to evaporate, and then using a scraper to scrape off the adhesive liquid that rises above the perforated mold 20. At this point, the adhesive liquid in the through holes 101 is already semi-solid adhesive bodies 14.

Optionally, the method of drying the adhesive liquid includes heating to dry or drying in the air, which can be freely chosen according to actual needs. In this embodiment, the core rod and the overall structure on the core rod are placed in an oven and dried at a temperature range of 60°C-300°C for 15-60 minutes. The sprayed surface is then checked for dryness, and after the adhesive liquid is dried, the perforated mold 20 is carefully removed to ensure that the adhesive bodies 14 in the through holes 101 are fixed on the outer surface of the inner membrane 11.

Optionally, when the vascular stent 13 is sleeved on the inner membrane 11, the vascular stent 13 is fixed according to positioning fines, ensuring that the adhesive bodies 14 are aligned with holes or gaps on the vascular stent 13, and ensuring that the adhesive bodies 14 are located at the positions having minimal influence to the expansion process of the vascular stent 13, the inner membrane 11 and the outer membrane 12. Preferably, the plurality of adhesive bodies 14 are distributed on the symmetry line of adjacent two ribs 131, or the plurality of adhesive bodies 14 are distributed adjacent to the symmetry line of adjacent two ribs 131.

Optionally, the heat shrinkable device can be a heat shrink tube or a heat treatment mold. In this embodiment, the heat shrink tube is wrapped around an outer surface of the outer membrane 12 to assist in heat treatment shaping. The heat shrink tube needs to be made of a material that can withstand at least 300°C, such as PTFE.

Optionally, the temperature for heating the overall structure on the core rod is 200°C-300°C, such as 220°C, 240°C, 260°C, 280°C; the time is 5-60 minutes, such as 10, 20, 30, 40, and 50 minutes. In this embodiment, the overall structure on the core rod is placed in a hot oven or a circulating hot air furnace for sintering.

Optionally, the inner membrane 11 and the outer membrane 12 are polymer membranes, such as ePTFE membranes.

Optionally, the inner membrane 11 and/or the outer membrane 12 is a tubular or sheet-like membrane, and the vascular stent 13 is located between the inner membrane 11 and the outer membrane 12.

Optionally, the vascular stent 13 is tubular, and the material of the vascular stent 13 is cobalt chromium alloy or stainless steel, but not limited to this.

Optionally, as shown in FIG. 3, FIG. 4, and FIG. 5, the vascular stent 13 further includes a plurality of connecting bars 132 connected between adjacent two ribs 131. The plurality of ribs 131 are spaced apart from each other along an axial direction of the covered stent 10, and each rib 131 is wrapped around the axis of the covered stent 10. When the vascular stent 13 expands, the connecting bars 132 are compressed by adjacent two ribs 131, and the connecting bars 132 can provide reverse support force to the adjacent ribs 131. Therefore, the vascular stent 13 can ensure a good support performance and a significant post expansion performance while reducing the axial shortening rate of the vascular stent 13 during expansion; especially, when the vascular stent 13 is covered by the inner membrane 11 and the outer membrane 12, it is more prone to shortening in the axial direction due to the binding effects of the membranes. The covered stent 10 made of this structure of the vascular stent 13 can effectively compensate for this binding defect.

Optionally, as shown in FIG. 4 and FIG. 5, the plurality of adhesive bodies 14 between adjacent two ribs 131 are arranged at intervals around the axis of the covered stent 10, that is, the plurality of adhesive bodies 14 are wrapped around the axis of the covered stent 10.

Optionally, as shown in FIG. 3 and FIG. 5, the wavy ribs 131 extend in a wave shape, a sawtooth shape, or a square wave shape.

Optionally, FIG. 6 is a schematic diagram of the unfolded state of the vascular stent and the adhesive bodies according to another embodiment of the present invention. As shown in FIG. 6, the rib 131 includes a plurality of bending units 201 connected to each other, and each bending unit 201 includes a first rib section 1311 and a second rib section 1312, which are arranged in mirror symmetry. When the vascular stent 13 is sleeved on the inner membrane 11, a plurality of adhesive bodies 14 are distributed on a symmetry line of the first rib section 1311 and the second rib section 1312, or a plurality of adhesive bodies 14 are distributed adjacent to a symmetry line of the first rib section 1311 and the second rib section 1312. The adhesive bodies 14 are located at the positions having minimal influence to the expansion process of the inner membrane 11 and the outer membrane 12. The adhesive bodies 14 can more firmly connect the inner membrane 11 with the outer membrane 12, making the shape of the overall membranes controllable, while ensuring the expansion elasticity of the material at the unconnected locations of the inner membrane 11 and the outer membrane 12.

Optionally, as shown in FIG. 5, the connecting bar 132 is in a straight strip shape, and the length direction of the connecting bar 132 is parallel to the axis of the covered stent 10.

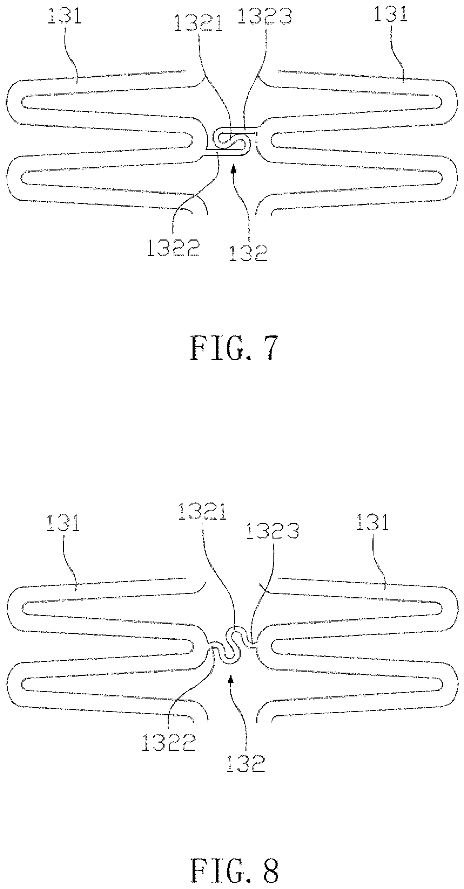

Optionally, FIG. 7 is a structural schematic diagram of the connecting bar according to the second embodiment of the present invention. As shown in FIG. 7, the connecting bar 132 includes a bending portion 1321, a first connecting portion 1322, and a second connecting portion 1323. The bending portion 1321 is bent in an S-shape, with one end of the bending portion 1321 connected to the first connecting portion 1322 and the other end of the bending portion 1321 connected to the second connecting portion 1323. The first connecting portion 1322 and the second connecting portion 1323 are respectively connected to adjacent two ribs 131. In this embodiment, the first connecting portion 1322 is parallel to the second connecting portion 1323, and the bending portion 1321 is connected between the first connecting portion 1322 and the second connecting portion 1323; preferably, the first connecting portion 1322 and the second connecting portion 1323 are parallel to the axis of the covered stent 10, or the first connecting portion 1322 and the second connecting portion 1323 are inclined relative to the axis of the covered stent 10.

Optionally, FIG. 8 is a structural schematic diagram of the connecting bar according to the third embodiment of the present invention. As shown in FIG. 8, the structure of the connecting bar 132 in this embodiment is basically the same as that of the connecting bar 132 in the second embodiment, with the difference in the orientation of the bending portion 1321.

Specifically, the direction of the bending portion 1321 in this embodiment is perpendicular to the direction of the bending portion 1321 in the second embodiment.

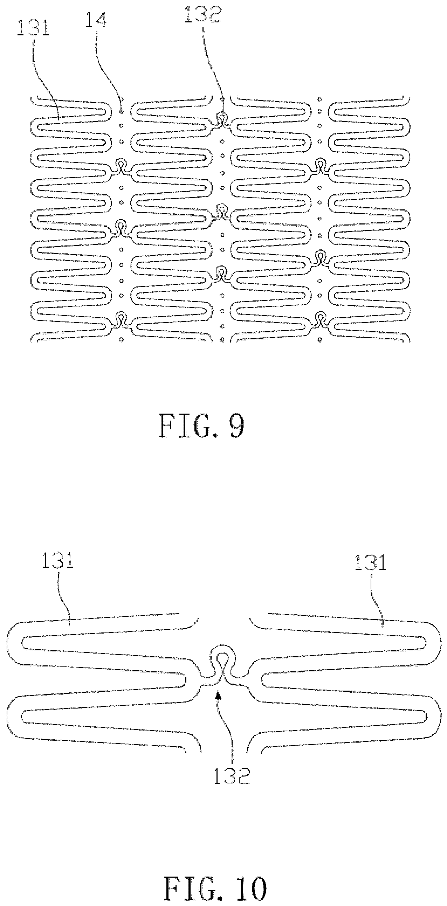

Optionally, FIG. 9 is a schematic diagram of the unfolded state of the vascular stent and the adhesive bodies according to the fourth embodiment of the present invention, and FIG. 10 is an enlarged schematic diagram of the connecting bar shown in FIG. 9. As shown in FIG. 9 and FIG. 10, the connecting bar 132 in this embodiment has a different structure from that in the second embodiment, and the connecting bar 132 in this embodiment is in the shape of a symbol Ω.

In other embodiments, the connecting bar 132 is in a wave shape, a sawtooth shape, a square wave shape, or a plate wave shape.

As shown in FIG. 3 and FIG. 4, the present invention further provides a covered stent 10, and the covered stent 10 is prepared using the method for manufacturing a covered stent described above.

Optionally, the covered stent 10 includes an inner membrane 11, an outer membrane 12, a vascular stent 13, and a plurality of adhesive bodies 14. The inner membrane 11 is located on an inner side of the vascular stent 13, and the outer membrane 12 is located on an outer side of the vascular stent 13. The vascular stent 13 is located between the inner membrane 11 and the outer membrane 12. The plurality of adhesive bodies 14 are arranged in a lattice pattern, one end of each adhesive body 14 is fixed to the inner membrane 11, and the other end of each adhesive body 14 is fixed to the outer membrane 12.

Optionally, the inner membrane 11 and/or the outer membrane 12 is a tubular or sheet-like membrane.

Optionally, the vascular stent 13 is tubular, and the material of the vascular stent 13 is cobalt chromium alloy or stainless steel, but not limited to this.

Optionally, as shown in FIG. 3 and FIG. 4, the vascular stent 13 includes a plurality of wavy ribs 131 and a plurality of connecting bars 132 connected between adjacent two ribs 131, and adjacent two ribs 131 are arranged in mirror symmetry. The plurality of ribs 131 are spaced apart from each other along an axial direction of the covered stent 10, and each rib 131 is wrapped around the axis of the covered stent 10. When the vascular stent 13 expands, the connecting bars 132 are compressed by adjacent two ribs 131, and the connecting bars 132 can provide reverse support force to the adjacent ribs 131. Therefore, the vascular stent 13 can ensure a good support performance and a significant post expansion performance, while reducing the axial shortening rate of the vascular stent 13 during expansion. Especially, when the vascular stent 13 is covered by the inner membrane 11 and the outer membrane 12, it is more prone to shortening in the axial direction due to the binding effects of the membranes. The covered stent 10 made of this structure of the vascular stent 13 can effectively compensate for this binding defect.

Optionally, as shown in FIG. 4 and FIG. 5, the plurality of adhesive bodies 14 in the gaps are arranged at intervals around the axis of the covered stent 10, that is, the plurality of adhesive bodies 14 are wrapped around the axis of the covered stent 10.

Optionally, the plurality of adhesive bodies 14 are located on the symmetry line of adjacent two ribs 131, or the plurality of adhesive bodies 14 are located adjacent to the symmetry line of adjacent two ribs 131.

Optionally, as shown in FIG. 6, the rib 131 includes a plurality of bending units 201 connected to each other. Each bending unit 201 includes a first rib section 1311 and a second rib section 1312, which are arranged in mirror symmetry. A plurality of adhesive bodies 14 are distributed on a symmetry line of the first rib section 1311 and the second rib section 1312, or a plurality of adhesive bodies 14 are arranged adjacent to a symmetry line of the first rib section 1311 and the second rib section 1312. The adhesive bodies 14 are located at the positions having minimal influence to the expansion process of the inner membrane 11 and the outer membrane 12. The adhesive bodies 14 can more firmly connect the inner membrane 11 with the outer membrane 12, making the shape of the overall membranes controllable, while ensuring the expansion elasticity of the material at the unconnected locations of the inner membrane 11 and the outer membrane 12.

Optionally, as shown in FIG. 3 and FIG. 5, the wavy ribs 131 extend in a wave shape, a sawtooth shape, or a square wave shape.

Optionally, as shown in FIG. 5, the connecting bar 132 is in a straight strip shape, and the length direction of the connecting bar 132 is parallel to the axis of the covered stent 10.

Optionally, as shown in FIG. 7, the connecting bar 132 includes a bending portion 1321, a first connecting portion 1322, and a second connecting portion 1323. The bending portion 1321 is bent in an S-shape, with one end of the bending portion 1321 connected to the first connecting portion 1322 and the other end of the bending portion 1321 connected to the second connecting portion 1323. The first connecting portion 1322 and the second connecting portion 1323 are respectively connected to adjacent two ribs 131. In this embodiment, the first connecting portion 1322 is parallel to the second connecting portion 1323, and the bending portion 1321 is connected between the first connecting portion 1322 and the second connecting portion 1323; preferably, the first connecting portion 1322 and the second connecting portion 1323 are parallel to the axis of the covered stent 10, or the first connecting portion 1322 and the second connecting portion 1323 are inclined relative to the axis of the covered stent 10.

Optionally, as shown in FIG. 8, the structure of the connecting bar 132 in this embodiment is basically the same as that of the connecting bar 132 in the second embodiment, with the difference in the orientation of the bending portion 1321.

Specifically, the direction of the bending portion 1321 in this embodiment is perpendicular to the direction of the bending portion 1321 in the second embodiment.

Optionally, as shown in FIG. 9 and FIG. 10, the structure of the connecting bar 132 in this embodiment is different from that of the connecting bar 132 in the second embodiment, and the connecting bar 132 in this embodiment is in the shape of a symbol Ω.

In other embodiments, the connecting bar 132 is in a wave shape, a sawtooth shape, a square wave shape, or a plate wave shape.

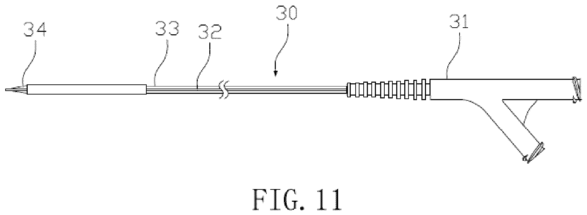

FIG. 11 is a structural schematic diagram of the covered stent delivery system according to the fifth embodiment of the present invention. As shown in FIG. 11, the covered stent delivery system 30 is used to deliver the above-mentioned covered stent 10 into a blood vessel. The covered stent delivery system 30 includes a connecting member 31, a wire guide tube 32, a liquid guide tube 33, a balloon 34, and a developing ring (not shown). A wire guide cavity and a liquid guide cavity are provided inside the connecting member 31. Both the wire guide tube 32 and the liquid guide tube 33 are connected to the connecting member 31. The wire guide tube 32 is communicated with the wire guide cavity, the liquid guide tube 33 is communicated with the liquid guide cavity, and the wire guide tube 32 is inserted into the liquid guide tube 33. One end of the wire guide tube 32 extends out from one end of the liquid guide tube 33. One end of the balloon 34 is connected to the wire guide tube 32, and the other end of the balloon 34 is connected to the liquid guide tube 33. The liquid guide tube 33 is communicated with the balloon 34. The developing ring is connected to the wire guide tube 32, and the covered stent 10 is installed on the balloon 34. In this embodiment, the wire guide tube 32 is used for guiding a guide wire, and the guide wire can pass through the wire guide cavity of the connecting member 31 and the interior of the wire guide tube 32; the liquid guide tube 33 is used to pass a liquid, and the liquid can enter the balloon 34 from the liquid guide cavity of the connecting member 31 and the liquid guide tube 33, forcing the balloon 34 to expand.

Optionally, the balloon 34 is located at the ends of the wire guide tube 32 and the liquid guide tube 33 away from the connecting member 31. One end of the balloon 34 is connected to the liquid guide tube 33 through laser welding, thermal welding, or adhesive bonding, while the other end of the balloon 34 is connected to the wire guide tube 32 through laser welding, thermal welding, or adhesive bonding.

Optionally, the balloon 34 is made of materials such as PET, PEBAX, PU, PA, etc., and is blow molded by a balloon molding machine.

Optionally, the diameter of the balloon 34 is 2-15mm, such as 4mm, 6mm, 8mm, 12mm; the length of the balloon 34 is 10-200mm, such as 20mm, 40mm, 60mm, 120mm, and 150mm.

Optionally, the wire guide tube 32 and/or the liquid guide tube 33 are made of materials such as PET, PEBAX, PU, PA, etc. through extrusion molding.

Optionally, the developing ring is a platinum iridium alloy ring, and is used for internal development.

The steps of delivering the covered stent 10 into a blood vessel by the covered stent delivery system 30 of the present invention include:
step 1: using conventional interventional surgical procedures to establish operational pathways such as vascular sheath, guide sheath, guide wire, etc., and determining the location of the lesion where the covered stent 10 should be placed.
step 2, using a balloon folding machine to fold the balloon 34 to a small diameter and make it shape memory.
step 3: using a stent holder to press the covered stent 10 tightly onto the balloon 34.
step 4: pushing the covered stent delivery system 30 forward along the guide wire already placed to the target position, and then filling the balloon 34 with a liquid through the liquid guide cavity and the liquid guide tube 33, causing the balloon 34 to expand, thereby driving the covered stent 10 to expand until it fits the blood vessel wall.
step 5: sucking out the liquid inside the balloon 34 through the liquid guide cavity and the liquid guide tube 33, causing the inflated balloon 34 to retract, and finally, retracting the covered stent delivery system 30. At this point, the covered stent 10 has been placed in the target position to complete the treatment task.

The above embodiments are only illustrative of the principles and effects of the present invention, and are not intended to limit the present invention. Anyone familiar with this technology may modify or alter the above embodiments without departing from the spirit and scope of the present invention. Therefore, all equivalent modifications or changes made by those with ordinary knowledge in the relevant technical field without departing from the spirit and technical ideas disclosed in the present invention should still be covered by the claims of the present invention.

### Industrial applicability

The method for manufacturing a covered stent of the present invention adopts a lattice arrangement of the adhesive bodies to connect the inner membrane and the outer membrane. The adhesive bodies are distributed on the symmetrical line of adjacent two ribs, or the adhesive bodies are distributed adjacent to the symmetrical line of adjacent two ribs. The adhesive bodies are located far away from the ribs. The adhesive bodies are located at the positions having minimal influence to the expansion process of the inner membrane and the outer membrane. The adhesive bodies can more firmly connect the inner membrane with the outer membrane, making the shape of the overall membranes controllable, while ensuring the expansion elasticity of the material at the unconnected locations of the inner membrane and the outer membrane. Moreover, the covered stent manufactured by the manufacturing method further includes a plurality of connecting bars connected between adjacent two ribs. When the vascular stent expands, the connecting bars are compressed by adjacent two ribs, and the connecting bars can provide reverse support force to the adjacent ribs. Therefore, the covered stent has the function of reducing axial shortening rate.

## Claims

1. A method for manufacturing a covered stent, wherein the method for manufacturing a covered stent comprises:
providing an inner membrane, and sleeving the inner membrane on a core rod;
providing a perforated mold, and fixing the perforated mold onto the inner membrane, wherein the perforated mold is provided with a plurality of through holes for exposing the inner membrane, and the plurality of through holes are arranged in a lattice pattern;
providing an adhesive liquid, and arranging the adhesive liquid in each of the through holes to form an adhesive body in each of the through holes;
removing the perforated mold to leave the adhesive bodies arranged in a lattice pattern on a surface of the inner membrane;
providing a vascular stent, wherein the vascular stent comprises a plurality of wavy ribs, and adjacent two ribs are arranged in mirror symmetry, and sleeving the vascular stent on the inner membrane, such that a plurality of adhesive bodies are distributed on a symmetry line of adjacent two ribs, or a plurality of adhesive bodies are distributed adjacent to a symmetry line of adjacent two ribs;
providing an outer membrane, and covering the outer membrane on the vascular stent;
providing a heat shrinkable device, and sleeving the heat shrinkable device on the outer membrane;
heating the overall structure on the core rod, such that the adhesive bodies are fixedly connected between the inner membrane and the outer membrane, and then removing the heat shrinkable device and the core rod to form a covered stent.

2. The method for manufacturing a covered stent as claimed in claim **1**, wherein before sleeving the inner membrane and/or the outer membrane on the core rod, the method comprises:
performing a surface treatment on an outer surface of the inner membrane and an inner surface of the outer membrane, wherein the surface treatment comprises any one or more than one of the following:
using a surface modifier containing boron and amino groups;
using an etching method with sodium naphthalene;
using a processing method with ArF laser;
using a chemical grafting method.

3. The method for manufacturing a covered stent as claimed in claim **1**, wherein the perforated mold is made of FEP membrane, steel strip, or steel pipe.

4. The method for manufacturing a covered stent as claimed in claim **1**, wherein the heat shrinkable device is a heat shrink tube or a heat treatment mold; the inner membrane and the outer membrane are ePTFE membranes.

5. The method for manufacturing a covered stent as claimed in claim **1**, wherein the adhesive liquid is a solution, a suspension, or an emulsion; the solute of the adhesive liquid includes granule or powder of polymer materials.

6. The method for manufacturing a covered stent as claimed in claim **5**, wherein the solvent of the adhesive liquid is a liquid that is volatile, has no chemical residue, and does not corrode the inner membrane and the outer membrane.

7. The method for manufacturing a covered stent as claimed in claim **5**, wherein the emulsion is dynamically stable for 10-60 minutes.

8. The method for manufacturing a covered stent as claimed in claim **1**, wherein the wavy ribs extend in a wave shape, a sawtooth shape, or a square wave shape.

9. The method for manufacturing a covered stent as claimed in claim **8**, wherein the rib comprises a plurality of bending units connected to each other, and each bending unit comprises a first rib section and a second rib section, which are arranged in mirror symmetry; when the vascular stent is sleeved on the inner membrane, a plurality of adhesive bodies are distributed on a symmetry line of the first rib section and the second rib section, or a plurality of adhesive bodies are distributed adjacent to a symmetry line of the first rib section and the second rib section.

10. The method for manufacturing a covered stent as claimed in any one of claims **1** to **9**, wherein the inner membrane and/or the outer membrane is a tubular or sheet-like membrane.

11. The method for manufacturing a covered stent as claimed in claim **10**, wherein the vascular stent further comprises a plurality of connecting bars connected between adjacent two ribs, the connecting bar is in a straight strip shape, a wave shape, a sawtooth shape, a square wave shape, a plate wave shape, or a symbol Ω.

12. The method for manufacturing a covered stent as claimed in claim **1**, wherein the method of drying the adhesive liquid comprises heating to dry or drying in the air, wherein the temperature of heating to dry is 60°C-300°C, and the drying time is 15-60 minutes.

13. The method for manufacturing a covered stent as claimed in claim **1**, wherein the temperature for heating the overall structure on the core rod is 200°C-300°C, and the time is 5-60 minutes.

14. A covered stent, wherein the covered stent is manufactured using the method for manufacturing a covered stent as claimed in any one of claims **1** to **13**, the covered stent comprises an inner membrane, an outer membrane, a vascular stent, and a plurality of adhesive bodies, the inner membrane is arranged on an inner side of the vascular stent, the outer membrane is arranged on an outer side of the vascular stent, the vascular stent is located between the inner membrane and the outer membrane, the plurality of adhesive bodies are arranged in a lattice pattern, one end of each adhesive body is fixed to the inner membrane, and the other end of each adhesive body is fixed to the outer membrane.

15. A covered stent delivery system, wherein the covered stent delivery system is used to deliver the covered stent as claimed in claim **14** into a blood vessel, the covered stent delivery system comprises a connecting member, a wire guide tube, a liquid guide tube, a balloon, and a developing ring, a wire guide cavity and a liquid guide cavity are provided inside the connecting member, both the wire guide tube and the liquid guide tube are connected to the connecting member, the wire guide tube is communicated with the wire guide cavity, the liquid guide tube is communicated with the liquid guide cavity, the wire guide tube is inserted into the liquid guide tube, one end of the wire guide tube extends out from one end of the liquid guide tube, one end of the balloon is connected to the wire guide tube, the other end of the balloon is connected to the liquid guide tube, the liquid guide tube is communicated with the balloon, the developing ring is connected to the wire guide tube, and the covered stent is installed on the balloon.
